# EUROPEAN PATENT SPECIFICATION

(11) **EP 0 580 723 B1**
(45) Date of publication and mention of the grant of the patent: **25.10.1995**
(21) Application number: 92909898.6
(22) Date of filing: 14.04.1992
(51) Int. Cl.: A61M 5/142

(54) **INFUSER**
INFUSIONSEINRICHTUNG
INFUSEUR

(30) Priority: 18.04.1991 DK 694/91
(43) Date of publication of application: 02.02.1994
(73) Proprietor: NOVO NORDISK A/S, 2880 Bagsvaerd (DK)
(72) Inventor: POULSEN, Jens, Ulrik, DK-2200 Copenhagen N (DK); PLUM, Thomas, Munk, DK-2942 Skodsborg (DK); MOLLER-JENSEN, Jens, DK-Copenhagen K (DK)
(74) Representative: Hansen, Einar Tronier
(86) International application number: DK9200125
(87) International publication number: WO9218175

(56) References cited:
- EP-A- 0 062 974
- EP-A- 0 275 213
- EP-A- 0 399 119
- WO-A-85/00523

## Description

The invention relates to pumps for current infusion of liquid, preferably insulin.

By insulin treatment of type 1 diabetes a syringe, a pen, or a pump are used.

The pump offers the possibility of a good controlling of the glucose concentration as it may simulate the course of the insulin production by a non-diabetic. However, the use of insulin pumps has been rather limited as pump treatment has been looked on as a strict treatment and as the pumps have been complex to operate.

Consequently, the basis for the invention is the wish of making it clear that an insulin pump allows a free life style with a good control of the glucose and may be very simple to use.
By integrating more disposable parts in a single unit the apparatus is made more simple to handle than apparatus wherein a cartridge, an infusion line, and batteries for the pumping motor must be changed individually.

From EP 62 974 is known an infusion pump comprising a permanent portion containing a battery, a programmable electronic controller, and pump drive means, and a disposable portion including a reservoir filled with a liquid medication to be dispensed, and a pump for pumping the medication from the reservoir into the user's body. The pump includes a pump chamber and a piston connected to an armature responding to the pump drive means. All the parts of the pump coming into contact with the liquid is comprised by the disposable portion.

From WO 85/00523, which is considered to represent the closest prior art with regard to the present invention, it is known to integrate the power source for the pump into a disposable part which further comprises an infusion line for insertion into a pump head to provide a peristaltic pump.

The invention provides a pump for infusing a liquid from a reservoir, comprising a durable part forming a housing and a disposable part containing the reservoir and an energy reservoir for energizing the pumping function, the disposable part further comprising all liquid contacting elements of a pump mechanism pumping the liquid and all liquid contacting elements of possible sensors measuring the pressure in an outlet from the pumping mechanism, and wherein the disposable part and the durable part are provided with mating coupling means, which pump according to the invention is characterized in, that the disposable part further comprises a memory keeping an account of the amount of liquid left in the reservoir.

Comprising such a memory the disposable part will always be able to provide a control unit with the correct information regarding the amount of liquid left in the reservoir without any manual setting or resetting have to be performed. Thereby safety and convenience is provided.

According to the invention the durable part may comprise one or more of the elements: A controlling unit, a display, means for setting the controlling unit, a drive unit delivering mechanical energy for driving the pump mechanism, and a long life electric cell energizing the controlling unit. When the controlling unit is energized by its own electric cell it is avoided that data stored in this unit are deleted during the change of the disposable part. It is possible to energize the controlling unit from the energy reservoir in the disposable part so that only during change the energizing is switched over to the long life cell in the durable part, whereby this cell only acts as an emergency power supply.

The means for setting the controlling unit may comprise a socket with electric contacts, which socket is designed to receive and communicate with a plug having corresponding electric contacts and carrying a programmed ROM-circuit defining the infusion data. The plug may further carry a graphic representation of the infusion data stored in the ROM, i.e. how the infusion of a set 24-hours' dose is distributed over the 24 hours. This graphic representation may be marks on a transparent sheet so secured to the plug that it covers a watch dial display when the plug is inserted in the socket, the marks indicating periods with increased or decreased infusion. By this construction the user may avoid the relatively complicated programming of the pump as he may plan the needed infusion profile or profiles in cooperation with his medical adviser, and thereafter he will only have to insert the plug which is in accordance with his immediate life style.

To establish the necessary communication between the durable and the disposable part, these parts are provided with cooperating sets of electric contacts and cooperating coupling parts so that electrical and mechanical connections are established concomitantly when the disposable part is mounted to the durable part.

The mounting may easily be made when the durable and the disposable parts in a complementary way fill the inner space of the housing whereby the disposable part is unambiguously secured in the housing.

The invention will now be explained in further details with reference to the drawing, in which
Fig. 1 shows the durable part of a pump according to the invention,
Fig. 2 shows the disposable part of a pump according to the invention,
Fig. 3 shows the disposable part of Fig. 2 mounted in the durable part, which is shown with its lid open, and
Fig. 4 shows an infusion data carrying plug with an attached information sheet.

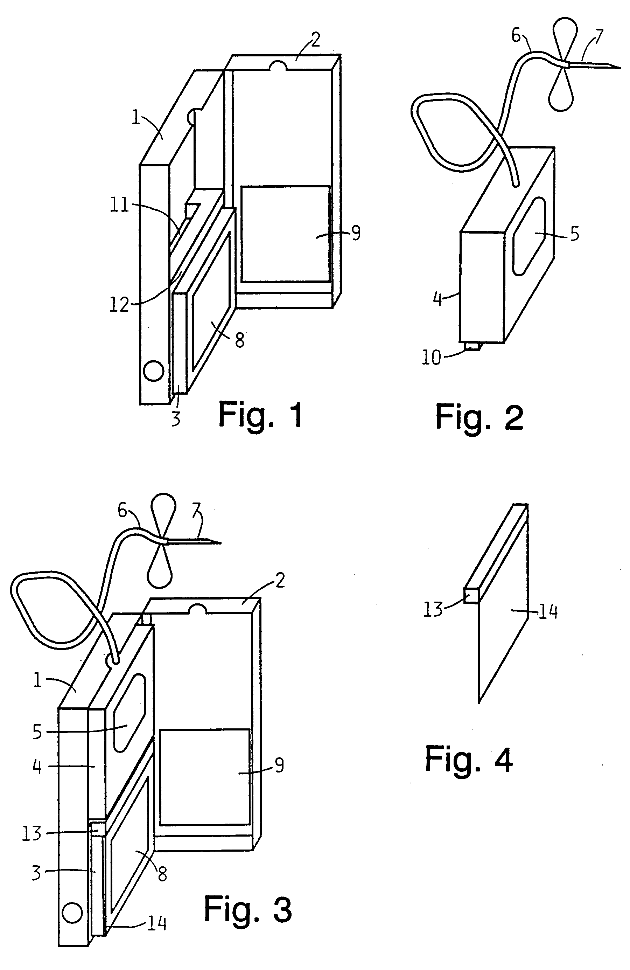

The infusion apparatus shown in Fig. 1 has a durable part forming a housing and comprising a bottom part 1 and a lid 2 hinged to this bottom part. In one end of the bottom part 1 there is an electronic compartment 3 comprising a controlling unit and a display 8.

In the other end of the bottom part 1 space is left for receiving a disposable part 4 as shown in Fig. 2. This disposable part contains an insulin reservoir made inspectable through a window 5. The disposable part further contains a pump mechanism for delivering the insulin through an infusion line 6 to a needle 7 for insertion in the tissue of the patient.

The energy for the pump is supplied by a battery in the disposable part. This battery is designed with a capacity sufficient to energize the pump during the emptying of the insulin reservoir.

In a preferred embodiment, the disposable part comprises a sensor placed at the inlet of the infusion line and designed to be coupled to a measuring device.

As it appears, all parts which have to be changed frequently are integrated in the disposable part. The time for changing is defined by the draining of the insulin reservoir and it is automatically ensured that also the battery and the infusion line are changed.

In this preferred embodiment, also members which are not normally changed so as the pump mechanism and the sensor are integrated in the disposable part.

Fig. 3 shows a pump with the disposable part mounted in the bottom part. By this mounting sets of electrical contacts 10 and 11 in the disposal and the durable part, respectively, establish electrical connections between the disposable part 4 and the durable part 3 which controls the infusion.

When a disposable part is mounted in the bottom part 1 of the housing, the lid 2 is closed and is kept closed by a locking mechanism. The lid 2 is provided with a window 9 which reveals the relevant part of the display when the lid 2 is closed.

In Figure 1 another set of electric contacts are provided in a socket 12 mating a linear plug 13 as shown in Figure 3. In the plug a ROM circuit is provided, in which circuit information is stored defining how a set 24-hours' dose should be distributed taking into account that the need for insulin varies during a day and a night depending on the patient's life style. This feature makes it easy for the patient to change the 24 hours' infusion profile in accordance with changes in life style, e.g. from working days to week-ends or holidays, just by inserting another plug 13 into the socket.

Figure 4 shows such a plug 13 being provided with a transparent sheet which is attached to the plug 13 so that it covers the display 8 of the durable part of the pump when the plug 13 is inserted into the socket 12 of this durable part. The sheet 14 may carry information of the infusion profile obtained with the current plug. The sheet being transparent the information may be graphic and may be seen in connection with information shown on the display 8.

The apparatus is described as a pump for infusing insulin, but may be used for other kinds of timed medication without deviating from the scope of the invention.

## Claims

1. A pump for infusing a liquid from a reservoir, comprising a durable part forming a housing (1,2) and a disposable part (4) containing the reservoir and an energy reservoir for energizing the pumping function, the disposable part (4) further comprising all liquid contacting elements of a pump mechanism pumping the liquid and all liquid contacting elements of possible sensors measuring the pressure in an outlet from the pumping mechanism, and wherein the disposable part (4) and the durable part are provided with mating coupling means (10,11), **characterized** in, that the disposable part (4) further comprises a memory keeping an account of the amount of liquid left in the reservoir

2. A pump according to claim 1, **characterized** in that the durable part comprises one or more of the elements: A controlling unit, a display (8), means for setting the controlling unit, a drive unit delivering mechanical energy for driving the pumping mechanism and a long life electric cell for energizing the control unit.

3. A pump according to claim 2, **characterized** in that the means for setting the controlling unit comprises a socket (12) with electric contacts, this socket being designed to receive and communicate with a plug (13) having corresponding electric contacts and carrying a programmed ROM-circuit defining the infusion data.

4. A pump according to claim 3, **characterized** in that the plug (13) further carries a graphic representation of the infusion data stored in the ROM.

5. A pump according to claim 4, **characterized** in that the graphic representation is provided as marks on a transparent sheet (14) secured to the plug (13) so that it covers a watch dial display on the durable display (8), when the plug (13) is inserted into the socket (12).

6. A pump according to any of the preceding claims, **characterized** in that the durable part and the disposable part (4) are provided with cooperating sets of electric contacts and cooperating coupling parts (10, 11) so that electric and mechanical connections are established concomitantly when the disposable part (4) is mounted to the durable part.

7. A pump according to any of the preceding claims, **characterized** in that the durable and the disposable parts in a complementary way fill the inner space of the housing (1, 2) whereby the disposable part (4) is unambiguously secured in the housing.

## Patentansprüche

1. Eine Pumpe zur Infusion einer Flüssigkeit aus einem Reservoir, die ein langlebiges Teil, das ein Gehäuse (1, 2) bildet, und ein Einwegteil (4) umfaßt, das das Reservoir und einen Energiespeicher zur Energieversorgung der Pumpfunktion enthält, wobei das Einwegteil (4) außerdem alle mit Flüssigkeit in Kontakt kommenden Elemente eines Pumpmechanismus, der die Flüssigkeit pumpt, und alle mit Flüssigkeit in Kontakt kommenden Elemente möglicher Sensoren, die den Druck in einem Auslaß aus dem Pumpmechanismus messen, umfaßt und wobei das Einwegteil (4) und das langlebige Teil mit zusammenpassenden Kupplungsmitteln (10, 11) versehen sind, dadurch gekennzeichnet, daß das Einwegteil (4) einen Speicher umfaßt, der Buch führt über die im Reservoir übriggebliebene Menge an Flüssigkeit.

2. Eine Pumpe nach Anspruch 1, dadurch gekennzeichnet, daß das langlebige Teil eines oder mehrere der Elemente umfaßt: Eine Steuereinheit, ein Display (8), Mittel zur Einstellung der Steuereinheit, eine Antriebseinheit, die mechanische Energie zum Antrieb des Pumpmechanismus liefert, und eine langlebige elektrische Zelle zur Energieversorgung der Steuereinheit.

3. Eine Pumpe nach Anspruch 2, dadurch gekennzeichnet, daß die Mittel zur Einstellung der Steuereinheit eine Buchse (12) mit elektrischen Kontakten umfaßt, wobei diese Buchse so konstruiert ist, daß sie einen Stecker (13) aufnimmt und mit diesem in Verbindung steht, der entsprechende elektrische Kontakte aufweist und eine programmierte ROM-Schaltung trägt, die die Infusionsdaten definiert.

4. Eine Pumpe nach Anspruch 3, dadurch gekennzeichnet, daß der Stecker (13) außerdem eine graphische Darstellung der im ROM gespeicherten Infusionsdaten trägt.

5. Eine Pumpe nach Anspruch 4, dadurch gekennzeichnet, daß die graphische Darstellung als Markierungen auf einem durchsichtigen Blatt (14) vorgesehen ist, das an dem Stecker (13) befestigt ist, so daß sie eine Uhren-Ziffernblatt-Anzeige auf dem langlebigen Display (8) überdeckt, wenn der Stecker (13) in die Buchse (12) eingeschoben ist.

6. Eine Pumpe nach einem der vorangehenden Ansprüche, dadurch gekennzeichnet, daß das langlebige Teil und das Einwegteil (4) mit zusammenwirkenden Sätzen von elektrischen Kontakten und zusammenwirkenden Kupplungsteilen (10, 11) versehen sind, so daß, elektrische und mechanische Verbindungen gleichzeitig hergestellt werden, wenn das Einwegteil (4) an dem langlebigen Teil angebracht wird.

7. Eine Pumpe nach einem der vorangehenden Anprüche, dadurch gekennzeichnet, daß die langlebigen und die Einwegteile in komplementärer Art und Weise den Innenraum des Gehäuses (1, 2) ausfüllen, wodurch das Einwegteil (4) unzweideutig im Gehäuse befestigt wird.

## Revendications

1. Pompe pour la perfusion d'un liquide à partir d'un réservoir, comprenant une partie durable formant un boîtier (1,2) et une partie jetable (4) contenant le réservoir et un réservoir d'énergie pour alimenter en courant la fonction de pompage, la partie jetable (4) comprenant en outre tous les éléments entrant en contact avec le liquide d'un mécanisme de pompe qui pompe le liquide, et tous les éléments entrant en contact avec le liquide d'éventuels capteurs mesurant la pression à la sortie du mécanisme de pompage, et dans laquelle la partie jetable (4) et la partie durable sont munies de systèmes de raccordement (10, 11), caractérisée en ce que la partie jetable (4) comprend en outre une mémoire qui comptabilise la quantité de liquide qui reste dans le réservoir.

2. Pompe selon la revendication 1, caractérisée en ce que la partie durable comprend un ou plusieurs des éléments suivants: une unité de commande, un visuel (8), un système de mise au point de l'unité de commande, une unité d'entraînement délivrant une énergie mécanique pour actionner le mécanisme de la pompe, et une cellule électrique de longue durée fournissant du courant à l'unité de commande.

3. Pompe selon la revendication 2, caractérisée en ce que le système de mise au point de l'unité de commande comprend une prise (12) avec des contacts électriques, cette prise étant destinée à recevoir une fiche (13) ayant des contacts électriques correspondants et portant un circuit de mémoire morte programmé définissant les données de la perfusion, et à communiquer avec cette fiche.

4. Pompe selon la revendication 3, caractérisée en ce que la fiche (13) porte en outre une représentation graphique des données de la perfusion stockées dans la mémoire morte.

5. Pompe selon la revendication 4, caractérisée en ce que la représentation graphique est fournie sous forme de marques sur une feuille transparente (14) fixée sur la fiche (13) de façon à recouvrir une visualisation de cadran de montre sur le visuel durable (8) lorsque la fiche (13) est insérée dans la prise (12).

6. Pompe selon l'une quelconque des revendications précédentes, caractérisée en ce que la partie durable et la partie jetable (4) sont munies de jeux de contacts électriques concourants et d'éléments de couplage concourants (10, 11) de façon que les raccordements électriques et mécaniques s'établissent simultanément lorsque l'on monte la partie jetable (4) sur la partie durable.

7. Pompe selon l'une quelconque des revendications précédentes, caractérisée en ce que les parties durable et jetable remplissent de façon complémentaire l'espace intérieur du boîtier (1, 2), grâce à quoi la partie jetable (4) est fixée sans équivoque dans le boîtier.
